# EUROPEAN PATENT SPECIFICATION

(11) **EP 1 945 818 B1**
(45) Date of publication and mention of the grant of the patent: **03.01.2018**
(21) Application number: 06837099.8
(22) Date of filing: 07.11.2006
(51) Int. Cl.: C12Q 1/68

(54) **CHLAMYDIA TRACHOMATIS SPECIFIC OLIGONUCLEOTIDE SEQUENCES**
CHLAMYDIA TRACHOMATIS SPEZIFISCHE OLIGONUKLEOTIDE
SÉQUENCES D'OLIGONUCLÉOTIDES SPECIFIQUES À CHLAMYDIA TRACHOMATIS

(30) Priority: 07.11.2005 US 734155 P
(43) Date of publication of application: 23.07.2008
(60) Divisional application: 17198310.9
(73) Proprietor: Siemens Healthcare Diagnostics Inc., Tarrytown, NY 10591 (US)
(72) Inventor: KU, Lailing, Pleasanton, CA 94588 (US); BUSH-DONOVAN, Charlene, Livermore, CA 94551 (US); SHERMAN, David, Davis, CA 95616 (US); MENG, Qi, Fremont, CA 94539 (US)
(74) Representative: Schweitzer, Klaus
(86) International application number: PCT/US2006/043394
(87) International publication number: WO 2007/056398

(56) References cited:
- US-A- 6 096 501
- US-B1- 6 218 125
- DATABASE EMBL [Online] 8 June 1988 (1988-06-08), "Chlamydia trachomatis cryptic plasmid pLGV440" XP002443137 retrieved from EBI accession no. EMBL:X06707 Database accession no. X06707 -& HATT ET AL: "Analysis of the entire nucleotide sequence of the cryptic plasmid C. trachomatis serovar L1" NUCLEIC ACIDS RESEARCH, OXFORD UNIVERSITY PRESS, SURREY, GB, vol. 16, no. 9, 1988, pages 4053-4067, XP002087847 ISSN: 0305-1048
- WESTIN L ET AL: "ANTIMICROBIAL RESISTANCE AND BACTERIAL IDENTIFICATON UTILIZING A MICROELECTRONIC CHIP ARRAY" JOURNAL OF CLINICAL MICROBIOLOGY, WASHINGTON, DC, US, vol. 39, no. 3, March 2001 (2001-03), pages 1097-1104, XP001006185 ISSN: 0095-1137
- SPEARS P A ET AL: "SIMULTANEOUS STRAND DISPLACEMENT AMPLIFICATION AND FLUORESCENCE POLARIZATION DETECTION OF CHLAMYDIA TRACHOMATIS DNA" ANALYTICAL BIOCHEMISTRY, ACADEMIC PRESS, SAN DIEGO, CA, US, vol. 247, 1997, pages 130-137, XP000764850 ISSN: 0003-2697

## Description

### Related Applications

This application claims priority from Provisional Application No. 60/734,155, filed on November 7, 2005 and entitled "Chlamydia Trachomatis Specific Oligonucleotide Sequences".

### Background of the Invention

Chlamydiae are widespread intracellular bacterial pathogens that are responsible for a wide variety of important human and animal infections. Chlamydiae are obligate, non-motile, gram-negative bacteria characterized by a unique biphasic life cycle with dimorphic forms that are functionally and morphologically different. *Chlamydia trachomatis,* one of the four main species of the *Chlamydiaceae* family, is almost exclusively a human pathogen, and is the world's most frequent cause of sexually transmitted disease and preventable blindness. *Chlamydia trachomatis* exists as 15 different serotypes, including serotypes A, B, Ba and C, which cause trachoma (a form of bilateral kerato-conjunctivitis, which afflicts over 400 million people primarily in Africa, the Middle East, and Asia); serotypes D to K, which are responsible for inclusion conjunctivitis and genital tract infections; and serotypes L1 to L3, which are associated with lymphogranuloma venereum (a sexually transmitted disease that is rare in the U.S. and Europe, but may account for 2-10% of patients with genital ulcer disease in tropical countries).

Genital chlamydial infection is the most common sexually transmitted disease (STD) in the western world, and the most prevalent bacterial STD in the United States. In 2002, more than 800,000 new cases of genital chlamydia were reported to the CDC, exceeding all other notifiable diseases in the U.S. (Centers for Disease Control and Prevention, *"Sexually Transmitted Disease Surveillance, 2002",* U.S. Department of Health and Human Services, Atlanta, GA, September 2003). Under-reporting is substantial because as many as 70-80% of infections in women and approximately 50% in men are clinically silent (H.D. Davies et al., CMAJ, 1996, 153: 1631-1644; S.D. Hillis et al., Sex. Transm. Dis., 1995, 22: 197-202; A.C. Gerbase et al., Sex. Transm. Infect., 1998, 74: S12-S14; A.C. Gerbase et al., Lancet, 1998, 351: 2-4). Unrecognized and untreated, the bacteria may remain infectious in the host for several months. The asymptomatic nature of *Chlamydia trachomatis* infection may facilitate its spread in the at-risk population and promote a reservoir of infection (S.E. Thompson and A.E. Washington, Epidemiol. Rev., 1983, 5: 96-123; T. Ripa, Scand. J. Infect. Dis. Suppl., 1990, 69: 157-167).

When they are present, symptoms of chlamydial infection in women include increased vaginal discharge, post-coital and/or inter-menstrual bleeding, lower abdominal pain and dysuria (B.A. Cromer and F.P. Head, Sex. Transm. Dis., 1987, 14: 125-129; S.M. Garland and B. Johnson, Med. J. Austral., 1989, 150: 174-177; G.R. Scott et al., Br. J. Obstet Gynaecol., 1989, 96: 473-477; C.K. Malotte et al., Am. Public Health, 1990, 80: 469-471; P. Oakeshott et al., Fam. Pract., 1992, 9: 421-424; J.T. Humphreys et al., Sex. Transm. Dis., 1992, 19: 47-53). Babies born to infected mothers are at risk for conjunctivitis and pneumonia. In men, symptoms include urethral discharge and dysuria (J. Schachter, N. Engl. J. Med., 1978, 298: 428-435). If untreated, chlamydial infections can progress to serious reproductive and other health problems, with both short-term and long-term consequences. Like the disease itself, the damage that Chlamydia causes is often "silent". In women, untreated infection can spread into the uterus and/or fallopian tubes, and cause pelvic inflammatory disease (N.S. Padian and A.E. Washington, Ann. Epidemiol., 1994, 4: 128-132). This happens in up to 40% of women with untreated Chlamydia. Pelvic inflammatory disease (PID) can cause permanent damage to the fallopian tubes, uterus, and surrounding tissues, which can lead to chronic pelvic pain, infertility, and potentially fatal ectopic pregnancy (W. Cates Jr. et al., Am. J. Obstet. Gynecol., 1991, 164: 1771-1781; J. Coste et al., Fertil. Steril., 1994, 62: 289-295; L. Weström and P. Wolner-Hansen, Genitourin. Med., 1993, 69: 9-17). In men, untreated chlamydia may lead to prostatitis, scarring of the urethra, infertility and epididymitis. Although patients with any sexually transmitted disease are at increased risk of co-infection with another STD, co-infection of chlamydia and gonorrhea is most common. Forty percent (40%) of women and 20% of men with chlamydial infection are co-infected with gonorrhea. Chlamydial infection has also been reported to be associated with an increased risk for development of cervical cancer (S. Hillis et al., Sex. Transm. Dis., 1995, 22: 197-202; T. Anttila et al., JAMA, 2001, 286: 47-51).

Since curative antibiotic therapy for chlamydial infections is readily available and inexpensive, early diagnosis is an essential component of public health programs to control these infections. The goals of early detection include interruption of the chain of transmission and prevention of long-term sequelae (J. Paavonen et al., Obstet. Gynecol., 1998, 92: 292-298). Patients with genital chlamydial infections are also at increased risk for infection with human immunodeficiency virus (HIV), if exposed. Shortening the duration of infectiousness by early diagnosis and treatment could have a major impact on risk reduction for HIV infection.

Isolation of *Chlamydia trachomatis* in cell culture has been the traditional method for laboratory diagnosis and has remained the method of choice for medico-legal specimens because of its specificity. However, this method requires expensive equipment, technical expertise, and stringent transport conditions to preserve specimen viability; it also has a turnaround time of 2 to 3 days. In many settings, cell culture has been replaced by more rapid tests based on antigen detection by direct fluorescent antibody staining, enzyme immunoassays, and enzyme-linked immunosorbent assays (ELISA), which have less demanding transport requirements and can provide results on the same day. However, these methods are still laborious and time-consuming and, more importantly, lack sensitivity as screening assays, especially for asymptomatic patients.

More recently, nucleic acid-based hybridization probe tests have been developed for direct detection of *Chlamydia trachomatis* (R. Warren et al., J. Clin. Microbiol., 1993, 31: 1663-1666). These tests offer higher specificity but no substantial improvement on sensitivity. Furthermore, most of these tests are performed on endocervical or urethral specimens, which are obtained using invasive sampling procedures. Nucleic acid amplification assays based on polymerase chain reaction (PCR), ligase chain reaction (LCR), strand-displacement amplification (SDA), branched DNA, or transcription-mediated amplification (TMA) technology are now available (M. Buimer et al., J. Clin. Microbiol., 1996, 34: 2395-2400; M.A. Chernesky et al., Mol. and Cell Probes, 1996, 11: 243-249; T.C. Quinn et al., J. Clin. Microbiol., 1996, 34: 1401-1406; G.L. Ridgway et al., J. Clin. Pathol., 1996, 49: 116-119; C.M. Black, Clin. Microbiol. Rev., 1997, 10: 160-184; K.A. Crotchfelt et al., J. Clin. Microbiol., 1997, 35: 1536-1540; P.O. Davies and G.L. Ridgway, Int. J. STD AIDS, 1997, 8: 731-738; L. Grun et al., NMJ, 1997, 315: 226-230; K.A. Crotchfelt et al., J. Clin. Microbiol., 1998, 36: 391-394; C.A. Gaydos et al., J. Infect. Dis., 1998, 177: 417-424; R. Pasternack et al., Eur. J. Clin. Microbiol. Infect. Dis., 1999, 18: 142-144). In addition to offering all the advantages of non-culture tests in terms of ambient specimen transport, batching automation, and rapid processing time, these assays provide higher specificity and a sensitivity approaching 100%. Furthermore, they can be performed on less invasive clinical specimens such as urine (J.E. Bauwens et al., J. Clin. Microbiol., 1993, 31: 3013-3116; M. Domeika et al., J. Clin. Microbiol., 1994, 32: 2350-2352; M.A. Chernesky et al., J. Clin. Microbiol., 1994, 32: 2682-2685; H.H. Lee et al., Lancet, 1995, 345: 213-216; M. Bassiri et al., J. Clin. Microbiol., 1995, 33: 898-900; T.C. Quinn et al., J. Clin. Microbiol., 1996, 34: 1401-1406; R. Pasternack et al., Eur. J. Clin. Microbiol. Infect. Dis., 1999, 18: 142-144; K. Templeton et al., Int. J. STD AIDS, 2001, 12: 793-796; R.A. McCartney et al., Br. J. Biomed. Sci., 2001, 58: 235-238; L.A. Cosentino et al., J. Clin. Microbiol., 2003, 41: 3592-3596). All these advantages make nucleic acid amplification assays particularly suited for detection of asymptomatic chlamydial infection and as a screening tool.

However, existing nucleic acid amplification assays for Chlamydia detection still exhibit certain disadvantages and limitations. Although these assays have been designed to minimize contamination, there is some reluctance to replace less sensitive tests with this relatively new technology. The primary concerns involve false-negative results caused by the presence of amplification inhibitors in certain specimens and false-positive results due to cross-contamination if strict quality control procedures are not applied. Other concerns include inability to detect all serotypes of *Chlamydia trachomatis* with equal efficiency, cost, and sample throughput. Clearly, the development of improved nucleic acid amplification assays for the detection of chlamydial infection remains highly desirable.

### Summary of the Invention

The present invention is directed to systems for the rapid, selective and specific detection of *Chlamydia trachomatis* in biological samples. In particular, the invention encompasses reagents that can be used for developing nucleic acid amplification tests for the detection and diagnosis of chlamydial infection. More specifically, the invention provides the use of an oligonucleotid defined by a nucleic acid sequence according to SEQ ID No: 1 for detecting Chlamydia trachomatis in a test sample,
wherein the step of detecting comprises contacting the test sample with the nucleic acid sequence according to SEQ ID NO: 1 such that the oligonucleotide can hybridize to the Chlamydia trachomatis nucleic acid and amplifying all or a portion of the Chlamydia trachomatis nucleic acid to obtain Chlamydia trachomatis amplicons, and detecting any Chlamydia trachomatis amplicons, wherein amplifying all or a portion of the Chlamydia trachomatis nucleic acid comprises submitting the test sample to a nucleic acid amplification reaction carried out under suitable amplification conditions and in the presence of suitable amplification reagents. The nucleotide according to SEQ ID No. 1 can be used as an amplification primer and detection probe for the detection of either strand of target nucleic acid sequences in the cryptic plasmid of *Chlamydia trachomatis.*

In certain embodiments, the oligonucleotide sequence is provided as primer sets and primer/probe sets that can be used in any of a variety of nucleic acid amplification assays including those involving real-time and multiplex detection.

The present invention also provides methods for detecting *Chlamydia trachomatis* in a test sample. Generally, such methods comprise contacting a test sample suspected of containing a *Chlamydia trachomatis* nucleic acid with the oligonucleotide according to SEQ ID No: 1 such that the oligonucleotide can hybridize to the *Chlamydia trachomatis* nucleic acid, if present in the sample; and detecting any oligonucleotide hybridized to the *Chlamydia trachomatis* nucleic acid, where the detection of an oligonucleotide hybridized to the *Chlamydia trachomatis* nucleic acid indicates the presence of *Chlamydia trachomatis* in the sample.

Other methods of the comprise contacting a test sample suspected of containing a *Chlamydia trachomatis* nucleic acid with the primer set or primer/probe set described herein and amplification reaction reagents to form a reaction mixture. The reaction mixture is then placed under amplification conditions so as to amplify the *Chlamydia trachomatis* nucleic acid, if present in the test sample, and generate an amplification product. The resulting amplification product may be detected using a variety of detection technologies. In certain embodiments, an amplification product/probe hybrid is formed using a detection probe of the present invention, and detection of such an hybrid indicates the presence of *Chlamydia trachomatis* in the test sample.

Additionally, the inventive oligonucleotide sequence for amplification primers and detection probes can be used in combination with other specific primers and probes in a nucleic acid amplification format for the simultaneous detection of *Chlamydia trachomatis* and other target organisms. In certain embodiments, the amplification primer and detection probes of the present invention are used in combination with *Neisseria gonorrhea* specific primers and probes for the simultaneous detection of *Chlamydia trachomatis* and *Neisseria gonorrhea.*

Kits comprising amplification primers and detection probes according to the present invention and, optionally, amplification reaction reagents, are also provided for the detection of chlamydial infection in test samples.

These and other objects, advantages and features of the present invention will become apparent to those of ordinary skill in the art having read the following detailed description of the preferred embodiments.

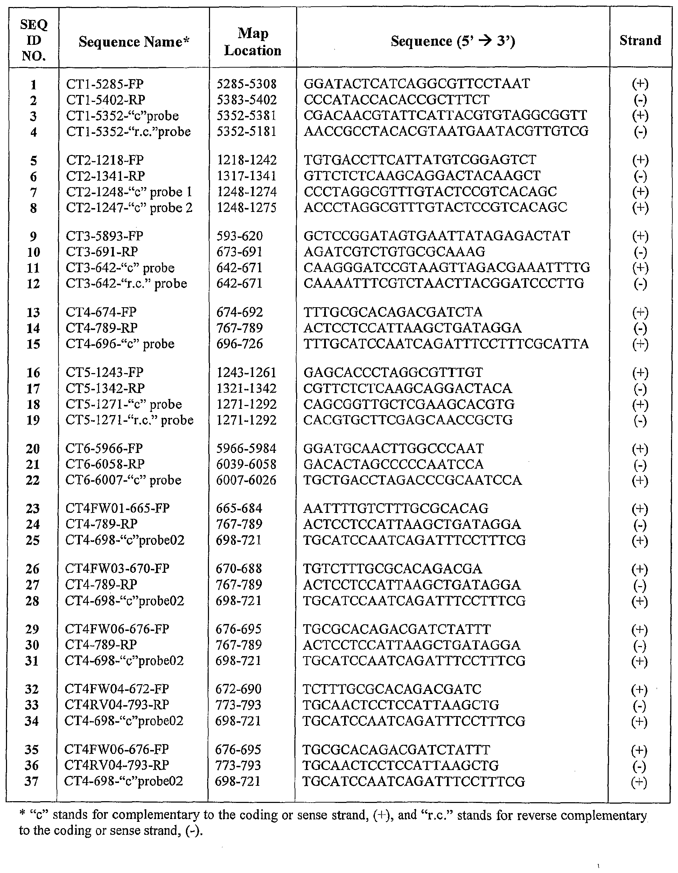

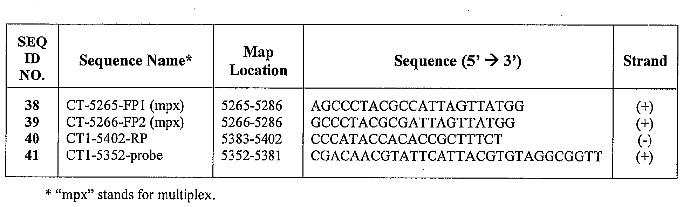

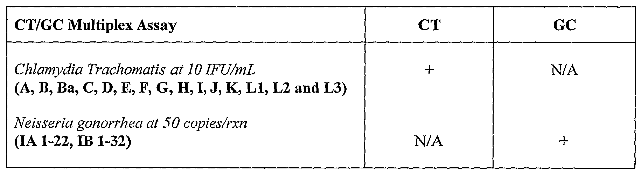

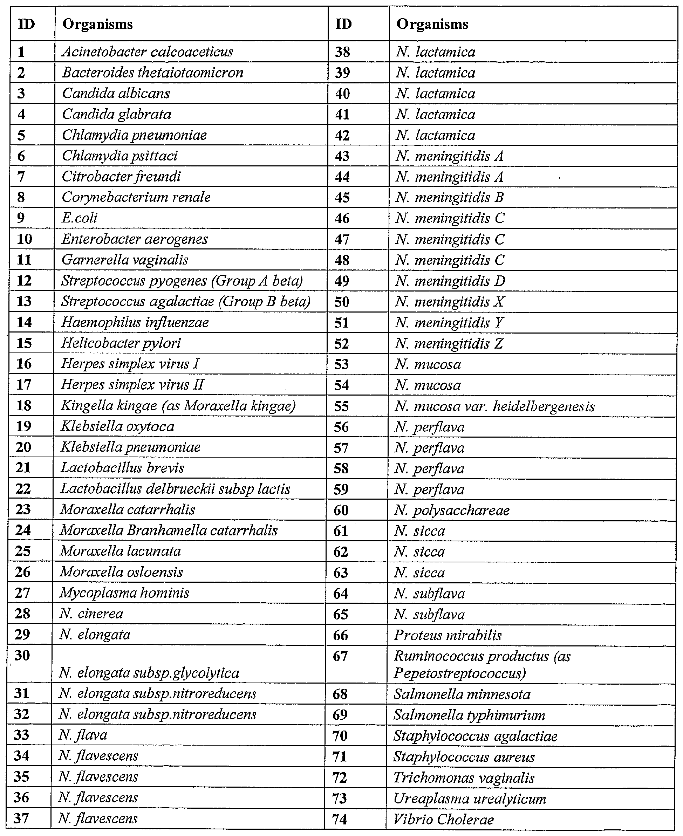

### Brief Description of the Drawing

**Table 1** shows the *Chlamydia trachomatis* specific amplification primer sequence SEQ ID No. 1, further amplification primer sequences that can be used in combination therewith and detection probe sequences derived from *Chlamydia trachomatis* cryptic plasmid L1 serovar. The map position and the SEQ ID NO. of the oligonucleotide are indicated in the table.
**Table 2** shows examples of *Chlamydia trachomatis* specific amplification primer sequences and detection probes that can be used in multiplex detection format assays.
**Table 3** shows the results of a multiplex TaqMan kPCR assay which was used to test fifteen (15) different *Chlamydia trachomatis* (CT) serovars and forty-six (46) different *Neisseria gonorrhea* (GC) isolates (see Example 1 for experimental details of the assay).
**Table 4** is a list of 74 closely related organisms, for which the CT/GC multiplex PCR master mix showed no cross-reactivity.

### Definitions

Throughout the specification, several terms are employed that are defined in the following paragraphs.

The terms ***"individual"*, *"subject"*** and ***"patient"*** are used herein interchangeably. They refer to a human being that can be the host of *Chlamydia trachomatis,* but may or may not be infected by the bacterium. The terms do not denote a particular age, and thus encompass adults, children, newborns, as well as fetuses.

The term ***"test sample",*** as used herein, refers to any liquid or solid material suspected of containing *Chlamydia trachomatis* nucleic acids. A test sample may be, or may be derived from, any biological tissue or fluid that can contain *Chlamydia trachomatis* nucleic acids. Frequently, the sample will be a "clinical sample", *i.e.,* a sample obtained or isolated from a patient to be tested for chlamydial infection. Such samples include, but are not limited to, bodily fluids which contain cellular materials and may or may not contain cells, *e.g.,* blood, plasma, serum, urine, seminal fluid, saliva, ocular lens fluid, lymphatic fluid, amniotic fluid, and the like; endocervical, urethral, rectal, vaginal, vulva-vaginal, nasopharyngeal and pulmonary samples; and archival samples with known diagnosis. Test samples may also include sections of tissues such as frozen sections. The term "test sample" also encompasses any material derived by processing a biological sample. Derived materials include, but are not limited to, cells (or their progeny) isolated from the sample, cell components, and nucleic acid molecules extracted from the sample. Processing of the biological sample to obtain a test sample may involve one or more of: filtration, distillation, centrifugation, extraction, concentration, dilution, purification, inactivation of interfering components, addition of reagents, and the like.

The terms ***"nucleic acid", "nucleic acid molecule"*** and ***"polynucleotide"*** are used herein interchangeably. They refer to a deoxyribonucleotide or ribonucleotide polymer in either single- or double-stranded form, and unless otherwise stated, encompass known analogs of natural nucleotides that can function in a similar manner as naturally-occurring nucleotides. The terms encompass nucleic acid-like structures with synthetic backbones, as well as amplification products.

The term ***"oligonucleotide",*** as used herein, refers to a short string of deoxyribonucleotide or ribonucleotide polymer that can be used as amplification primers or detection probes. These short stretches of nucleic acid sequences are often chemically synthesized, however, they can be prepared by any other suitable method. As will be appreciated by those skilled in the art, the length of an oligonucleotide (*i.e.,* the number of nucleotides) can vary widely, often depending on its intended function or use. Generally, oligonucleotides comprise between 5 and 300 nucleotides, for example between about 15 and about 100 nucleotides or between about 15 and about 50 nucleotides.

The term ***"isolated"*** when referring to an oligonucleotide means an oligonucleotide, which by virtue of its origin or manipulation, is separated from at least some of the components with which it is naturally associated. By "isolated", it is alternatively or additionally meant that the oligonucleotide of interest is produced or synthesized by the hand of man.

The term ***"active fragment",*** as used herein in reference to an oligonucleotide (*e.g.,* an oligonucleotide sequence provided herein), refers to any nucleic acid molecule comprising a nucleotide sequence sufficiently homologous to or derived from the nucleotide sequence of the oligonucleotide, which includes fewer nucleotides than the full length oligonucleotide, and retains at least one biological property of the entire sequence. Typically, active fragments comprise a sequence with at least one activity of the full length oligonucleotide. An active fragment or portion of an oligonucleotide sequence of the present invention can be a nucleic acid molecule which is, for example, 10, 15, 20, 25, 30 or more nucleotides in length and can be used as amplification primer and/or detection probe for the detection of *Chlamydia trachomatis* in a biological sample.

The term ***"sufficiently homologous",*** when used herein in reference to an active fragment of an oligonucleotide, refers to a nucleic acid molecule that has a sequence homology of at least 35% compared to the oligonucleotide. In certain embodiments, the sequence homology is at least 40%, at least 50%, at least 60%, at least 70%, at least 80%, at least 90% or at least 95%.

The terms ***"homology"*** and ***"identity"*** are used herein interchangeably, and refer to the sequence similarity between two nucleic acid molecules. Calculations of the percent homology or identity of two nucleic acid sequences, can be performed by aligning the two sequences for optimal comparison purposes (*e.g*., gaps can be introduced in one or both of a first and a second nucleic acid sequence for optimal alignment and non-homologous sequences can be disregarded for comparison purposes). In certain embodiments, the length of a sequence aligned for comparison purposes is at least 30%, at least 40%, at least 50%, at least 60%, at least 70%, a least 80%, at least 90%, at least 95% or 100% of the length of the reference sequence. The nucleotides at corresponding nucleotide positions are then compared. When a position in the first sequence is occupied by the same nucleotide as the corresponding position in the second sequence, then the molecules are identical (or homologous) at that position. The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. For example, the percent identity between two nucleotide sequences can be determined using the algorithm of Meyers and Miller (CABIOS, 1989, 4: 11-17), which has been incorporated into the ALIGN program (version 2.0), using a PAM120 weight residue table, a gap length penalty of 12 and a gap penalty of 4. The percent identity between two nucleotide sequences can, alternatively, be determined using the GAP program in the GCG software package (available at http://www.gcg.com), using a NWSgapdna.CMP matrix.

The term ***"hybridization"*** refers to the formation of complexes between nucleotide sequences which are sufficiently complementary to form complexes *via* Watson-Crick base pairing or non-canonical base pairing. When a primer "hybridizes" with a target sequence (template), such complexes (or hybrids) are sufficiently stable to serve the priming function required by, *e.g.,* the DNA polymerase, to initiate DNA synthesis. It will be appreciated that hybridizing sequences need not have perfect complementarity to provide stable hybrids. In many situations, stable hybrids will form where fewer than about 10% of the bases are mismatches. Accordingly, as used herein, the term ***"complementary"*** refers to an oligonucleotide that forms a stable duplex with its complement under assay conditions, generally where there is about 90% or greater homology. Those skilled in the art understand how to estimate and adjust the stringency of hybridization conditions such that sequences having at least a desired level of complementarity will stably hybridize, while those having lower complementarity will not. For examples of hybridization conditions and parameters see, *e.g.,* J. Sambrook et al., "Molecular Cloning: A Laboratory Manual", 1989, Second Edition, Cold Spring Harbor Press: Plainview, NY; F.M. Ausubel, "Current Protocols in Molecular Biology", 1994, John Wiley & Sons: Secaucus, NJ.

As used herein, the term ***"amplification"*** refers to a method that increases the representation of a population of specific nucleic acid sequences in a sample. Amplification methods (such as polymerase chain reaction or PCR) are known in the art and are discussed in more detail below.

As used herein, the term ***"target sequence"*** refers to a particular nucleic acid sequence which is to be detected. Preferably, target sequences include nucleic acid sequences to which oligonucleotide primers will complex. A target sequence may also include a probe-hybridizing region with which a probe will form a stable hybrid under desired conditions. As will be recognized by one of ordinary skill in the art, a target sequence may be single-stranded or double-stranded. In the context of the present invention, target sequences of interest are within the cryptic plasmid of *Chlamydia trachomatis.*

The terms ***"primer"*** and ***"amplification primer"*** are used herein interchangeably. They refer to an oligonucleotide which is capable of acting as a point of initiation of synthesis of a primer extension product, when placed under suitable conditions (*e.g*., buffer, salt, temperature and pH), in the presence of nucleotides and an agent for nucleic acid polymerization (*e.g*., a DNA-dependent or RNA-dependent polymerase). The primer is preferably single-stranded for maximum efficiency in amplification, but may alternatively be double-stranded. If double-stranded, the primer may first be treated (*e.g*., denatured) to allow separation of its strands before being used to prepare extension products. Such a denaturation step is typically performed using heat, but may alternatively be carried out using alkali, followed by neutralization. A typical primer contains about 10 to about 35 nucleotides in length of a sequence substantially complementary to the target sequence. However, a primer can also contain additional sequences. For example, amplification primers used in Strand Displacement Amplification (SDA) preferably include a restriction endonuclease recognition at site 5' to the target binding sequence (see, for example, U.S. Pat. Nos. 5,270,184 and 5,455,166). Nucleic Acid Sequence Based Amplification (NASBA), Self Sustaining Sequence Replication (3SR), and Transcription-Mediated Amplification (TMA) primers preferably include an RNA polymerase promoter linked to the target binding sequence of the primer. Methods for linking such specialized sequences to a binding target sequence for use in a selected amplification reaction are well-known in the art.

The terms **"*forward primer"*** and **"*forward amplification primer"*** are used herein interchangeably, and refer to a primer that hybridizes (or anneals) with the target sequence 5' with respect to the reverse primer. The terms ***"reverse primer"*** and ***"reverse amplification reverse primer"*** are used herein interchangeably, and refer to a primer that hybridizes (or anneals) to the target sequence 3' with respect to the forward primer.

The term ***"amplification conditions",*** as used herein, refers to conditions that promote annealing and/or extension of primer sequences. Such conditions are well-known in the art and depend on the amplification method selected. Thus, for example, in a PCR reaction, amplification conditions generally comprise thermal cycling, *i.e.,* cycling of the reaction mixture between two or more temperatures. In isothermal amplification reactions, amplification occurs without thermal cycling although an initial temperature increase may be required to initiate the reaction. Amplification conditions encompass all reaction conditions including, but not limited to, temperature and temperature cycling, buffer, salt, ionic strength, and pH, and the like.

As used herein, the term ***"amplification reaction reagents",*** refers to reagents used in nucleic acid amplification reactions and may include, but are not limited to, buffers, enzymes having reverse transcriptase and/or polymerase activity or exonuclease activity; enzyme cofactors such as magnesium or manganese; salts; nicotinamide adenine dinuclease (NAD); and deoxynucleoside triphosphates (dNTPs) such as deoxyadenosine triphospate, deoxyguanosine triphosphate, deoxycytidine triphosphate and thymidine triphosphate. Amplification reaction reagents may readily be selected by one skilled in the art depending on the amplification method used.

The terms ***"probe"*** and ***"detection probe"*** are used herein interchangeably and refer to an oligonucleotide capable of selectively hybridizing to at least a portion of a target sequence under appropriate conditions. In general, a probe sequence is identified as being either ***"complementary"*** to the coding or sense strand, or ***"reverse complementary"*** to the coding or sense strand. In certain preferred embodiments, a detection probe is labeled with a detectable moiety.

The terms ***"labeled"*** and ***"labeled with a detectable agent*** (or ***moiety***)" are used herein interchangeably to specify that an entity (*e.g.,* an oligonucleotide detection probe) can be visualized, for example following binding to another entity (*e.g.,* an amplification reaction product or amplicon). Preferably, the detectable agent or moiety is selected such that it generates a signal which can be measured and whose intensity is related to (*e.g*., proportional) the amount of bound entity. A wide variety of systems for labeling and/or detecting nucleic acid molecules are well-known in the art. Labeled nucleic acids can be prepared by incorporation of, or conjugation to, a label that is directly or indirectly detectable by spectroscopic, photochemical, biochemical, immunochemical, electrical, optical or chemical means. Suitable detectable agents include, but are not limited to, radionuclides, fluorophores, chemiluminescent agents, microparticles, enzymes, colorimetric labels, magnetic labels, haptens, Molecular Beacons, and aptamer beacons.

The terms ***"fluorophore",* "*fluorescent moiety",*** and **"*fluorescent dye"*** are used herein interchangeably. They refer to a molecule that absorbs a quantum of electromagnetic radiation at one wavelength, and emits one or more photons at a different, typically longer, wavelength in response. Numerous fluorescent dyes of a wide variety of structures and characteristics are suitable for use in the practice of the invention. Methods and materials are known for fluorescently labeling nucleic acid molecules (see, for example, R.P. Haugland, "Molecular Probes: Handbook of Fluorescent Probes and Research Chemicals 1992-1994", 5th Ed., 1994, Molecular Probes, Inc.). Preferably, a fluorescent moiety absorbs and emits light with high efficiency (*i.e.,* it has a high molar absorption coefficient at the excitation wavelength used, and a high fluorescence quantum yield), and is photostable (*i.e.,* it does not undergo significant degradation upon light excitation within the time necessary to perform the analysis). Rather than being directly detectable themselves, some fluorescent dyes transfer energy to another fluorescent dye in a process of fluorescent resonance energy transfer (FRET), and the second dye produces the detected signal. Such FRET fluorescent dye pairs are also encompassed by the term "fluorescent moiety". The use of physically linked fluorescent reporter/quencher moiety is also within the scope of the invention. In these embodiments, when the fluorescent reporter and quencher moiety are held in close proximity, such as at the ends of a nucleic acid probe, the quencher moiety prevents detection of a fluorescent signal from the reporter moiety. When the two moieties are physically separated, such as, for example, after cleavage by a Taq DNA polymerase, the fluorescent signal from the reporter moiety becomes detectable.

The term ***"directly detectable",*** when used herein in reference to a label or detectable moiety, means that the label or detectable moiety does not require further reaction or manipulation to be detectable. For example, a fluorescent moiety is directly detectable by fluorescence spectroscopy methods. The term ***"indirectly detectable",*** when used herein in reference to a label or detectable moiety, means that the label or detectable moiety becomes detectable after further reaction or manipulation. For example, a hapten becomes detectable after reaction with an appropriate antibody attached to a reporter, such as a fluorescent dye.

### Detailed Description of Certain Preferred Embodiments

As mentioned above, the present invention relates to methods and reagents for specifically and selectively detecting *Chlamydia trachomatis* in biological samples. *Chlamydia trachomatis* specific oligonucleotide sequences and sensitive nucleic acid amplification-based techniques are used that allow detection of *Chlamydia trachomatis* in samples containing even small amounts of the bacterium.

### I - Oligonucleotide Sequences for Amplification Primers and Detection Probes

### Oligonucleotide Sequences

In one aspect, the present invention provides an oligonucleotide sequence according to SEQ ID No. 1 that can be used in nucleic acid amplification tests for the specific detection of either strand of target sequences in the cryptic plasmid of *Chlamydia trachomatis.*

The 7,493 basepair cryptic plasmid of *Chlamydia trachomatis* is a good target for DNA-based diagnosis of chlamydial infection as it is specific to the organism and is found in essentially all clinical strains in approximately 7-10 copies per genome (J.E. Tam et al., Plasmid, 1992, 27: 231-236). All plasmids from human *Chlamydia trachomatis* isolates are extremely similar, with less than 1% nucleotide sequence variation. The entire DNA content of the cryptic plasmid of *Chlamydia trachomatis* has been sequenced (K.S. Sriprakash and E.S. Macavoy, Plasmid, 1987, 18: 205-214; C. Hatt et al., Nucleic Acids Res., 1988, 16: 4053-4067; M. Comanducci et al., Mol. Microbiol., 1988, 2: 531-538; M. Comanducci et al., Plasmid, 1990, 23: 149-154; N.S. Thomas and I.N. Clarke, Proc. 2nd Meeting Eur. Soc. Chlamydia Res. 1992, p. 42, Societa Editrice Esculapio: Bologna, Italy) and has been deposited in GenBank (Accession # X06707).

The oligonucleotide sequence used in the present invention is specific for target sequences in the cryptic plasmid of *Chlamydia trachomatis.* More specifically, the present invention provides the use of an oligonucleotide sequence for amplification primers and detection probes which recognize one or more serotypes of *Chlamydia trachomatis.* In certain embodiments, the inventive oligonucleotide sequences recognize all fifteen serotypes of *Chlamydia trachomatis.* Exemplary oligonucleotide sequences are presented in Table 1 and Table 2 (SEQ. ID NOs. 1 to 41), along with their corresponding map position. These sequences were identified by the present Applicants by sequence alignment with *Chlamydia trachomatis* cryptic plasmid pLGV440 sequence using Vector NTI, ABI primer express, and Oligo6 software programs.

As will be appreciated by one skilled in the art, any of the oligonucleotide sequences (or active fragments thereof) disclosed herein for amplification, detection or quantitation of *Chlamydia trachomatis* may be employed either as detection probes or amplification primers, depending on the intended use or assay format. For example, the inventive oligonucleotide sequence according to SEQ ID No. 1 used as an amplification primer in one assay can be used as a detection probe in another assay. A given sequence may be modified, for example, by attaching to the inventive oligonucleotide sequences, a specialized sequence (*e.g.,* a promoter sequence) required by the selected amplification method, or by attaching a fluorescent dye to facilitate detection. It is also to be understood that an oligonucleotide according to the present invention may include one or more sequences which can serve as spacers, linkers, sequences for labeling or binding to an enzyme, sequences which may impart added stability or susceptibility to degradation process or other desirable property to the oligonucleotide.

Based on the oligonucleotide sequences, one or more oligonucleotide analogues can be prepared (see below). Such analogues may contain alternative structures such as peptide nucleic acids or "PNAs" (*i.e.,* molecules with a peptide-like backbone instead of the phosphate sugar backbone of naturally occurring nucleic acids) and the like. Similarly, it is understood that oligonucleotides consisting of the sequences of the present invention may contain deletions, additions and/or substitutions of nucleic acid bases, to the extent that such alterations do not negatively affect the properties of the nucleic acid molecules. In particular, the alterations should not result in significant lowering of the hybridizing properties of the oligonucleotides.

### Primer Sets and Primer/Probe Sets

In another aspect, the present invention relates to combinations of oligonucleotide sequences disclosed herein for the detection of *Chlamydia trachomatis* in biological samples. More specifically, the present invention provides primer sets and primer/probe sets.

As used herein, the term ***"primer set"*** refers to two or more primers which together are capable of priming the amplification of a nucleotide sequence of interest (*e.g.,* a target sequence within the cryptic plasmid of *Chlamydia trachomatis*). In certain embodiments, the term "primer set" refers to a pair of primers including a forward primer and reverse primer. Such primer sets or primer pairs are particularly useful in PCR amplification reactions.

Examples of primer sets comprising a forward amplification primer and a reverse amplification primer include:
Primer Set 1, which comprises a forward primer comprising SEQ. ID NO. 1 (5'-GGATACTCATCAGGCGTTCCTAAT-3') or any active fragment thereof, and a reverse primer comprising SEQ. ID NO. 2 (5'-CCCATACCACACCGCTTTCT-3') or any active fragment thereof;
In the present invention the primer sets can be used in a multiplex format assay.

These primer sets can be used according to any nucleic acid amplification technique that employs two or more oligonucleotides to amplify a target sequence (as discussed below). Amplification products generated using the inventive primer sets may be detected using a variety of detection methods well known in the art. For example, amplification products may be detected using agarose gel electrophoresis and visualization by ethidium bromide staining and exposure to ultraviolet (UV) light or by sequence analysis of the amplification product for confirmation of *Chlamydia trachomatis* identity.

Alternatively, probe sequences can be employed using a variety of homogeneous or heterogeneous methodologies to detect amplification products. Generally in all such methods, the probe hybridizes to a strand of an amplification product (or amplicon) to form an amplification product/probe hybrid. The hybrid can then be directly or indirectly detected, for example using labels on the primers, probes or both the primers and probes.

As used herein, the term **"*primer*/*probe set"*** refers to a combination comprising two or more primers which together are capable of priming the amplification of a nucleotide sequence of interest (*e.g.,* a target sequence within the cryptic plasmid of *Chlamydia trachomatis*), and at least one probe which can detect the target sequence. The probe can hybridize to a strand of an amplification product (or amplicon) to form an amplification product/probe hybrid to allow detection of amplicons.

Accordingly, the present invention provides primer/probe sets that can be used according to nucleic acid amplification procedures to specifically amplify and detect *Chlamydia trachomatis* target sequences in test samples. The inventive primer/probe sets comprise a primer set, as described above, and at least one detection probe. The detection probe may comprise a detectable moiety. In certain embodiments, the detection probe comprises a fluorescent moiety attached at the 5' end and a quencher moiety attached at the 3' end.

Examples of primer/probe sets include:
Primer/Probe Set CT1, which comprises a forward primer comprising SEQ. ID NO. 1 (5'-GGATACTCATCAGGCGTTCCTAAT-3') or an active fragment thereof, a reverse primer comprising SEQ. ID NO. 2 (5'-CCCATACCACACCGCTTTCT-3') or an active fragment thereof, a complementary detection probe comprising SEQ. ID NO. 3 (5'-GACAACGTATTCATTACGTGTAGGCGGTT-3') or an active fragment thereof, and a reverse complementary detection probe comprising SEQ. ID NO. 4 (5'-AACCGCCTACACGTAATGAATACGTTGTCG-3') or an active fragment thereof;

In the present invention the primer/probe sets can be used in a multiplex detection format.

### Oligonucleotide Preparation

Oligonucleotides used in the invention may be prepared by any of a variety of methods (see, for example, J. Sambrook et al., "Molecular Cloning: A Laboratory Manual", 1989, 2nd Ed., Cold Spring Harbour Laboratory Press: New York, NY; *"*PCR Protocols: A Guide to Methods and Applications", 1990, M.A. Innis (Ed.), Academic Press: New York, NY; P. Tijssen "Hybridization with Nucleic Acid Probes - Laboratory Techniques in Biochemistry and Molecular Biology (Parts I and II)", 1993, Elsevier Science; *"*PCR Strategies", 1995, M.A. Innis (Ed.), Academic Press: New York, NY; and *"*Short Protocols in Molecular Biology", 2002, F.M. Ausubel (Ed.), 5th Ed., John Wiley & Sons: Secaucus, NJ). For example, the oligonucleotides may be prepared using any of a variety of chemical techniques well-known in the art, including, for example, chemical synthesis and polymerization based on a template as described, for example, in S.A. Narang et al., Meth. Enzymol. 1979, 68: 90-98; E.L. Brown et al., Meth. Enzymol. 1979, 68: 109-151; E.S. Belousov et al., Nucleic Acids Res. 1997, 25: 3440-3444; D. Guschin et al., Anal. Biochem. 1997, 250: 203-211; M.J. Blommers et al., Biochemistry, 1994, 33: 7886-7896; and K. Frenkel et al., Free Radic. Biol. Med. 1995, 19: 373-380; and U.S. Pat. No. 4,458,066).

For example, oligonucleotides may be prepared using an automated, solid-phase procedure based on the phosphoramidite approach. In such a method, each nucleotide is individually added to the 5'-end of the growing oligonucleotide chain, which is attached at the 3'-end to a solid support. The added nucleotides are in the form of trivalent 3'-phosphoramidites that are protected from polymerization by a dimethoxytriyl (or DMT) group at the 5' position. After base-induced phosphoramidite coupling, mild oxidation to give a pentavalent phosphotriester intermediate and DMT removal provides a new site for oligonucleotide elongation. The oligonucleotides are then cleaved off the solid support, and the phosphodiester and exocyclic amino groups are deprotected with ammonium hydroxide. These syntheses may be performed on oligo synthesizers such as those commercially available from Perkin Elmer/Applied Biosystems, Inc. (Foster City, CA), DuPont (Wilmington, DE) or Milligen (Bedford, MA). Alternatively, oligonucleotides can be custom made and ordered from a variety of commercial sources well-known in the art, including, for example, the Midland Certified Reagent Company (Midland, TX), ExpressGen, Inc. (Chicago, IL), Operon Technologies, Inc. (Huntsville, AL), and many others.

Purification of oligonucleotides of the invention, where necessary or desired, may be carried out by any of a variety of methods well-known in the art. Purification of oligonucleotides is typically performed either by native acrylamide gel electrophoresis, by anion-exchange HPLC as described, for example, by J.D. Pearson and F.E. Regnier (J. Chrom., 1983, 255: 137-149) or by reverse phase HPLC (G.D. McFarland and P.N. Borer, Nucleic Acids Res., 1979, 7: 1067-1080).

The sequence of an oligonucleotide can be verified using any suitable sequencing method including, but not limited to, chemical degradation (A.M. Maxam and W. Gilbert, Methods of Enzymology, 1980, 65: 499-560), matrix-assisted laser desorption ionization time-of-flight (MALDI-TOF) mass spectrometry (U. Pieles et al., Nucleic Acids Res., 1993, 21: 3191-3196), mass spectrometry following a combination of alkaline phosphatase and exonuclease digestions (H. Wu and H. Aboleneen, Anal. Biochem., 2001, 290: 347-352), and the like.

As already mentioned above, modified oligonucleotides may be prepared using any of several means known in the art. Non-limiting examples of such modifications include methylation, "caps", substitution of one or more of the naturally-occurring nucleotides with an analog, and internucleotide modifications such as, for example, those with uncharged linkages (*e.g*., methyl phosphonates, phosphotriesters, phosphoroamidates, carbamates, etc), or charged linkages (*e.g*., phosphorothioates, phosphorodithioates, etc). Oligonucleotides may contain one or more additional covalently linked moieties, such as, for example, proteins (*e.g.,* nucleases, toxins, antibodies, signal peptides, poly-L-lysine, etc), intercalators (*e.g.,* acridine, psoralen, etc), chelators (*e.g*., metals, radioactive metals, iron, oxidative metals, etc), and alkylators. The oligonucleotide may also be derivatized by formation of a methyl or ethyl phosphotriester or an alkyl phosphoramidate linkage. Furthermore, the oligonucleotide sequences of the present invention may also be modified with a label.

### Labeling of Oligonucleotide Sequences

In certain embodiments, detection probes or amplification primers or both probes and primers are labeled with a detectable agent or moiety before being used in amplification/detection assays. In certain embodiments, the detection probes are labeled with a detectable agent. The role of a detectable agent is to allow visualization and detection of amplified target sequences. Preferably, the detectable agent is selected such that it generates a signal which can be measured and whose intensity is related (*e.g*., proportional) to the amount of amplification products in the sample being analyzed.

The association between the oligonucleotide and the detectable agent can be covalent or non-covalent. Labeled detection probes can be prepared by incorporation of or conjugation to a detectable moiety. Labels can be attached directly to the nucleic acid sequence or indirectly (*e.g*., through a linker). Linkers or spacer arms of various lengths are known in the art and are commercially available, and can be selected to reduce steric hindrance, or to confer other useful or desired properties to the resulting labeled molecules (see, for example, E.S. Mansfield et al., Mol. Cell Probes, 1995, 9: 145-156).

Methods for labeling nucleic acid molecules are well-known in the art. For a review of labeling protocols, label detection techniques, and recent developments in the field, see, for example, L.J. Kricka, Ann. Clin. Biochem. 2002, 39: 114-129; R.P. van Gijlswijk et al., Expert Rev. Mol. Diagn. 2001, 1: 81-91; and S. Joos et al., J. Biotechnol. 1994, 35: 135-153. Standard nucleic acid labeling methods include: incorporation of radioactive agents, direct attachments of fluorescent dyes (L.M. Smith et al., Nucl. Acids Res., 1985, 13: 2399-2412) or of enzymes (B.A. Connoly and O. Rider, Nucl. Acids. Res., 1985, 13: 4485-4502); chemical modifications of nucleic acid molecules making them detectable immunochemically or by other affinity reactions (T.R. Broker et al., Nucl. Acids Res. 1978, 5: 363-384; E.A. Bayer et al., Methods of Biochem. Analysis, 1980, 26: 1-45; R. Langer et al., Proc. Natl. Acad. Sci. USA, 1981, 78: 6633-6637; R.W. Richardson et al., Nucl. Acids Res. 1983, 11: 6167-6184; D.J. Brigati et al., Virol. 1983, 126: 32-50; P. Tchen et al., Proc. Natl Acad. Sci. USA, 1984, 81: 3466-3470; J.E. Landegent et al., Exp. Cell Res. 1984, 15: 61-72; and A.H. Hopman et al., Exp. Cell Res. 1987, 169: 357-368); and enzyme-mediated labeling methods, such as random priming, nick translation, PCR and tailing with terminal transferase (for a review on enzymatic labeling, see, for example, J. Temsamani and S. Agrawal, Mol. Biotechnol. 1996, 5: 223-232). More recently developed nucleic acid labeling systems include, but are not limited to: ULS (Universal Linkage System), which is based on the reaction of monoreactive cisplatin derivatives with the N7 position of guanine moieties in DNA (R.J. Heetebrij et al., Cytogenet. Cell. Genet. 1999, 87: 47-52), psoralen-biotin, which intercalates into nucleic acids and upon UV irradiation becomes covalently bonded to the nucleotide bases (C. Levenson et al., Methods Enzymol. 1990, 184: 577-583; and C. Pfannschmidt et al., Nucleic Acids Res. 1996, 24: 1702-1709), photoreactive azido derivatives (C. Neves et al., Bioconjugate Chem. 2000, 11: 51-55), and DNA alkylating agents (M.G. Sebestyen et al., Nat. Biotechnol. 1998, 16: 568-576).

Any of a wide variety of detectable agents can be used in the practice of the present invention. Suitable detectable agents include, but are not limited to, various ligands, radionuclides (such as, for example, ³²P, ³⁵S, ³H, ¹⁴C, ¹²⁵I, ¹³¹I, and the like); fluorescent dyes (for specific exemplary fluorescent dyes, see below); chemiluminescent agents (such as, for example, acridinium esters, stabilized dioxetanes, and the like); spectrally resolvable inorganic fluorescent semiconductor nanocrystals (*i.e.,* quantum dots), metal nanoparticles (*e.g.,* gold, silver, copper and platinum) or nanoclusters; enzymes (such as, for example, those used in an ELISA, *i.e.,* horseradish peroxidase, beta-galactosidase, luciferase, alkaline phosphatase); colorimetric labels (such as, for example, dyes, colloidal gold, and the like); magnetic labels (such as, for example, Dynabeads™); and biotin, dioxigenin or other haptens and proteins for which antisera or monoclonal antibodies are available.

In certain preferred embodiments, the inventive detection probes are fluorescently labeled. Numerous known fluorescent labeling moieties of a wide variety of chemical structures and physical characteristics are suitable for use in the practice of this invention. Suitable fluorescent dyes include, but are not limited to, fluorescein and fluorescein dyes (*e.g*., fluorescein isothiocyanine or FITC, naphthofluorescein, 4',5'-dichloro-2',7'-dimethoxy-fluorescein, 6-carboxyfluorescein or FAM), carbocyanine, merocyanine, styryl dyes, oxonol dyes, phycoerythrin, erythrosin, eosin, rhodamine dyes (*e.g*., carboxytetramethylrhodamine or TAMRA, carboxyrhodamine 6G, carboxy-X-rhodamine (ROX), lissamine rhodamine B, rhodamine 6G, rhodamine Green, rhodamine Red, tetramethylrhodamine or TMR), coumarin and coumarin dyes (*e.g*., methoxy-coumarin, dialkylaminocoumarin, hydroxycoumarin and aminomethylcoumarin or AMCA), Oregon Green Dyes (*e.g.,* Oregon Green 488, Oregon Green 500, Oregon Green 514), Texas Red, Texas Red-X, Spectrum Red™, Spectrum Green™, cyanine dyes (*e.g.,* Cy-3™, Cy-5™, Cy-3.5™, Cy-5.5™), Alexa Fluor dyes (*e.g.,* Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), BODIPY dyes (*e.g.,* BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665), IRDyes (*e.g.,* IRD40, IRD 700, IRD 800), and the like. For more examples of suitable fluorescent dyes and methods for linking or incorporating fluorescent dyes to nucleic acid molecules see, for example, *"*The Handbook of Fluorescent Probes and Research Products", 9th Ed., Molecular Probes, Inc., Eugene, OR. Fluorescent dyes as well as labeling kits are commercially available from, for example, Amersham Biosciences, Inc. (Piscataway, NJ), Molecular Probes Inc. (Eugene, OR), and New England Biolabs Inc. (Berverly, MA).

Rather than being directly detectable themselves, some fluorescent groups (donors) transfer energy to another fluorescent group (acceptor) in a process of fluorescent resonance energy transfer (FRET), and the second group produces the detected fluorescent signal. In these embodiments, the oligonucleotide detection probe may, for example, become detectable when hybridized to an amplified target sequence. Examples of FRET acceptor/donor pairs suitable for use in the present invention include, but are not limited to fluorescein/tetramethylrhodamine, IAEDANS/FITC, IAEDANS/5-(iodoacetomido)fluorescein, EDANS/Dabcyl, and B-phycoerythrin/Cy-5.

The use of physically linked fluorescent reporter/quencher molecule pairs is also within the scope of the invention. The use of such systems in TaqMan™ assays (as described, for example, in U.S. Pat. Nos. 5,210,015; 5,804,375; 5487,792 and 6214,979) or as Molecular Beacons (as described, for example in, S. Tyagi and F.R. Kramer, Nature Biotechnol. 1996, 14: 303-308; S. Tyagi et al., Nature Biotechnol. 1998, 16: 49-53; L.G. Kostrikis et al., Science, 1998, 279: 1228-1229; D.L. Sokol et al., Proc. Natl. Acad. Sci. USA, 1998, 95: 11538-11543; S.A. Marras et al., Genet. Anal. 1999, 14: 151-156; and U.S. Pat. Nos. 5,846,726, 5,925,517, 6,277,581 and 6,235,504) is well-known in the art. With the TaqMan™ assay format, products of the amplification reaction can be detected as they are formed or in a so-called "real-time" manner. As a result, amplification product/probe hybrids are formed and detected while the reaction mixture is under amplification conditions.

In certain preferred embodiments of the present invention, the PCR detection probes are TaqMan™-like probes that are labeled at the 5'-end with a fluorescent moiety and at the 3'-end with a quencher moiety. Suitable fluorophores and quenchers for use with TaqMan™-like probes are disclosed, for example, in U.S. Pat. Nos. 5,210,015, 5,804,375, 5,487,792 and 6,214,979 and WO 01/86001. Examples of quenchers include, but are not limited to DABCYL (*i.e.,* 4-(4'-dimethylaminophenylazo)-benzoic acid) succinimidyl ester, diarylrhodamine carboxylic acid, succinimidyl ester (or QSY-7), and 4',5'-dinitrofluorescein carboxylic acid, succinimidyl ester (or QSY-33) (all available, for example, from Molecular Probes), quencher1 (Q1; available from Epoch Biosciences, Bothell, WA), or "Black hole quenchers" BHQ-1, BHQ-2, and BHQ-3 (available from BioSearch Technologies, Inc., Novato, CA). In certain embodiments of the present invention, the PCR detection probes are TaqMan™-like probes that are labeled at the 5' end with FAM and at the 3' end with a Black Hole Quencher.

A "tail" of normal or modified nucleotides can also be added to oligonucleotide probes for detectability purposes. A second hybridization with nucleic acid complementary to the tail and containing one or more detectable labels (such as, for example, fluorophores, enzymes or bases that have been radioactivity labeled) allows visualization of the amplicon/probe hybrids (see, for example, the system commercially available from Enzo Biochem. Inc., New York: NY). Another example of an assay with which the inventive oligonucleotides are useful is a signal amplification method such as that described in U.S. Pat. No. 5,124,246 (which is incorporated herein by reference in its entirety). In that method, the signal is amplified through the use of amplification multimers, polynucleotides which are constructed so as to contain a first segment that hybridizes specifically to the "tail" added to the oligonucleotide probes, and a multiplicity of identical second segments that hybridize specifically to a labeled probe. The degree of amplification is theoretically proportional to the number of iterations of the second segment. The multimers may be either linear or branched. Branched multimers may be in the shape of a fork or a comb.

The selection of a particular nucleic acid labeling technique will depend on the situation and will be governed by several factors, such as the ease and cost of the labeling method, the quality of sample labeling desired, the effects of the detectable moiety on the hybridization reaction (*e.g.,* on the rate and/or efficiency of the hybridization process), the nature of the amplification method used, the nature of the detection system, the nature and intensity of the signal generated by the detectable label, and the like.

### Amplification of Chlamydia trachomatis Target Sequences Using Inventive Primers

The use of oligonucleotide sequences of the present invention to amplify *Chlamydia trachomatis* target sequences in test samples is not limited to any particular nucleic acid amplification technique or any particular modification thereof. In fact, the inventive oligonucleotide sequences can be employed in any of a variety of nucleic acid amplification methods well-known in the art (see, for example, A.R. Kimmel and S.L. Berger, Methods Enzymol. 1987, 152: 307-316; J. Sambrook et al., "Molecular Cloning: A Laboratory Manual", 1989, 2nd Ed., Cold Spring Harbour Laboratory Press: New York, NY; *"*Short Protocols in Molecular Biology", F.M. Ausubel (Ed.), 2002, 5th Ed., John Wiley & Sons: Secaucus, NJ).

Such well-known nucleic acid amplification methods include, but are not limited to the Polymerase Chain Reaction (or PCR, described in, for example, *"*PCR Protocols: A Guide to Methods and Applications", M.A. Innis (Ed.), 1990, Academic Press: New York; *"*PCR Strategies", M.A. Innis (Ed.), 1995, Academic Press: New York; *"*Polymerase chain reaction: basic principles and automation in PCR: A Practical Approach", McPherson et al. (Eds.), 1991, IRL Press: Oxford; Saiki et al., Nature, 1986, 324: 163; and U.S. Pat. Nos. 4,683,195, 4,683,202 and 4,889,818, each of which is incorporated herein by reference in its entirety); and variations thereof including TaqMan™-based assays (Holland et al., Proc. Natl. Acad. Sci., 1991, 88: 7276-7280), and reverse transcriptase polymerase chain reaction (or RT-PCR, described in, for example, U.S. Pat. Nos. 5,322,770 and 5,310,652).

In PCR, a pair of primers is employed in excess to hybridize to the complementary strands of the target nucleic acid. The primers are each extended by a DNA polymerase using the target sequence as a template. The extension products become target themselves after dissociation (denaturation) from the original target strand. New primers are then hybridized and extended by the polymerase, and the cycle is repeated to exponentially increase the number of copies of target sequence molecules. Examples of DNA polymerases capable of producing primer extension products in PCR reactions include, but are not limited to: *E. coli* DNA polymerase I, Klenow fragment of DNA polymerase I, T4 DNA polymerase, thermostable DNA polymerases isolated from *Thermus aquaticus* (Taq), available from a variety of sources (for example, Perkin Elmer), *Thermus thermophilus* (United States Biochemicals), *Bacillus stereothermophilus* (Bio-Rad), or *Thermococcus litoralis* ("Vent" polymerase, New England Biolabs). RNA target sequences may be amplified by reverse transcribing the mRNA into cDNA, and then performing PCR (RT-PCR), as described above. Alternatively, a single enzyme may be used for both steps as described in U.S. Pat. No. 5,322,770.

In addition to the enzymatic thermal amplification described above, well-known isothermal enzymatic amplification reactions can be employed to amplify *Chlamydia trachomatis* target sequences using the oligonucleotide primers of the present invention (S.C. Andras et al., Mol. Biotechnol., 2001, 19: 29-44). These methods include, but are not limited to, Transcription-Mediated Amplification (or TMA, described in, for example, D.Y. Kwoh et al., Proc. Natl. Acad. Sci. USA, 1989, 86: 1173-1177; C. Giachetti et al., J. Clin. Microbiol., 2002, 40: 2408-2419; and U.S. Pat. No. 5,399,491); Self-Sustained Sequence Replication (or 3SR, described in, for example, J.C. Guatelli et al., Proc. Natl. Acad. Sci. USA, 1990, 87: 1874-1848; and E. Fahy et al., PCR Methods and Applications, 1991, 1: 25-33); Nucleic Acid Sequence Based Amplification (or NASBA, described in, for example, T. Kievits et al., J. Virol., Methods, 1991, 35: 273-286; and U.S. Pat. No. 5,130,238) and Strand Displacement Amplification (or SDA, described in, for example, G.T. Walker et al., PNAS, 1992, 89: 392-396; EP 0 500 224 A2). Each of the references cited in this paragraph is incorporated herein by reference in its entirety.

Strand-displacement amplification (SDA) combines the ability of a restriction endonuclease to nick the unmodified strand of its target DNA and the action of an exonuclease-deficient DNA polymerase to extend the 3' end at the nick and displace the downstream DNA strand at a fixed temperature (G.T. Walker et al., Proc. Natl. Acad. Sci. USA, 1992, 89: 392-396). Primers used in SDA include a restriction endonuclease recognition at site 5' to the target binding sequence (U.S. Pat. Nos. 5,270,184 and 5,344,166, each of which is incorporated herein by reference in its entirety).

Nucleic Acid Sequence Based Amplification (NASBA) uses three enzymes - *e.g*., RNase H, avian myeloblastosis virus (AMV) reverse transcriptase and T7 RNA polymerase - working in concert at a low isothermal temperature, generally 41°C (J. Compton, Nature, 1991, 350: 91-92; A.B. Chan and J.D. Fox, Rev. Med. Microbiol., 1999, 10: 185-196). The product of a NASBA reaction is mainly single-stranded RNA. The Self Sustaining Sequence Replication (3SR) reaction is a very efficient method for isothermal amplification of target DNA or RNA sequences. A 3SR system involves the collective activities of AMV reverse transcriptase, *E. Coli* RNase H, and DNA-dependent RNA polymerase (*e.g*., T7 RNA polymerase). Transcription-Mediated Amplification (TMA) uses an RNA polymerase to make RNA from a promoter engineered in the primer region, a reverse transcriptase to produce complementary DNA from the RNA templates and RNase H to remove the RNA from cDNA (J.C. Guatelli et al., Proc. Natl. Acad. Sci. USA, 1990, 87: 1874-1878).

NASBA, 3SR, and TMA primers require an RNA polymerase promoter linked to the target binding sequence of the primer. Promoters or promoter sequences for incorporation in the primers are nucleic acid sequences (either naturally occurring, produced synthetically or a product of a restriction digest) that are specifically recognized by an RNA polymerase that recognizes and binds to that sequence and initiates the process of transcription whereby RNA transcripts are generated. Examples of useful promoters include those which are recognized by certain bacteriophage polymerases such as those from bacteriophage T3, T7 or SP6 or a promoter from *E. coli.*

### Detection of Amplified Chlamydia trachomatis Target Sequences

In certain embodiments of the present invention, oligonucleotide probe sequences are used to detect amplification products generated by the amplification reaction (*i.e.,* amplified *Chlamydia trachomatis* target sequence). The inventive probe sequences can be employed using a variety of well-known homogeneous or heterogeneous methodologies.

Homogeneous detection methods include, but are not limited to, the use of FRET labels attached to the probes that emit a signal in the presence of the target sequence, Molecular Beacons (S. Tyagi and F.R. Kramer, Nature Biotechnol. 1996, 14: 303-308; S. Tyagi et al., Nature Biotechnol. 1998, 16: 49-53; L.G. Kostrikis et al., Science, 1998, 279: 1228-1229; D.L. Sokol et al., Proc. Natl. Acad. Sci. USA, 1998, 95: 11538-11543; S.A. Marras et al., Genet. Anal. 1999, 14: 151-156; and U.S. Pat. Nos. 5,846,726, 5,925,517, 6,277,581 and 6,235,504), and so-called TaqMan™ assays (U.S. Pat. Nos. 5,210,015; 5,804,375; 5487,792 and 6214,979 and WO 01/86001). Using these detection techniques, products of the amplification reaction can be detected as they are formed or in a so-called real time manner. As a result, amplification product/probe hybrids are formed and detected while the reaction mixture is under amplification conditions.

In certain preferred embodiments, the detection probes of the present invention are used in a TaqMan™ assay. A TaqMan™ assay, also known as fluorogenic 5' nuclease assay, is a powerful and versatile PCR-based detection system for nucleic acid targets. Analysis is performed in conjunction with thermal cycling by monitoring the generation of fluorescence signals. The assay system has the capability of generating quantitative data allowing the determination of target copy numbers. For example, standard curves can be produced using serial dilutions of previously quantified suspensions of *Chlamydia trachomatis,* against which sample unknowns can be compared. The TaqMan™ assay is conveniently performed using, for example, AmpliTaq Gold™ DNA polymerase, which has endogenous 5' nuclease activity, to digest an oligonucleotide probe labeled with both a fluorescent reporter dye and a quencher moiety, as described above. Assay results are obtained by measuring changes in fluorescence that occur during the amplification cycle as the probe is digested, uncoupling the fluorescent and quencher moieties and causing an increase in the fluorescence signal that is proportional to the amplification of the target sequence.

Other examples of homogeneous detection methods include hybridization protection assays (HPA). In such assays, the probes are labeled with acridinium ester (AE), a highly chemiluminescent molecule (Weeks et al., Clin. Chem., 1983, 29: 1474-1479; Berry et al., Clin. Chem., 1988, 34: 2087-2090), using a non-nucleotide-based linker arm chemistry (U.S. Pat. Nos. 5,585,481 and 5,185,439). Chemiluminescence is triggered by AE hydrolysis with alkaline hydrogen peroxide, which yields an excited N-methyl acridone that subsequently deactivates with emission of a photon. In the absence of a target sequence, AE hydrolysis is rapid. However, the rate of AE hydrolysis is greatly reduced when the probe is bound to the target sequence. Thus, hybridized and un-hybridized AE-labeled probes can be detected directly in solution, without the need for physical separation.

Heterogeneous detection systems are well-known in the art and generally employ a capture agent to separate amplified sequences from other materials in the reaction mixture. Capture agents typically comprise a solid support material (*e.g*., microtiter wells, beads, chips, and the like) coated with one or more specific binding sequences. A binding sequence may be complementary to a tail sequence added to the oligonucleotide probes of the invention. Alternatively, a binding sequence may be complementary to a sequence of a capture oligonucleotide, itself comprising a sequence complementary to a tail sequence of an inventive oligonucleotide probe. After separation of the amplification product/probe hybrids bound to the capture agents from the remaining reaction mixture, the amplification product/probe hybrids can be detected using any detection methods described above.

### II - Methods of Detection of Chlamydia trachomatis in Test Samples

In another aspect, the present invention provides methods for detecting the presence of *Chlamydia trachomatis* in a test sample. The inventive methods may be used, for example, to test patients who may or may not exhibit symptoms of chlamydial infection or its sequelae, and/or to screen at-risk populations.

Typically, methods of the invention comprise steps of: providing a test sample suspected of containing a *Chlamydia trachomatis* nucleic aid (*e.g.,* a nucleic acid comprising a sequence within the cryptic plasmid of *Chlamydia trachomatis*); contacting the test sample with at least one oligonucleotide disclosed herein, such that the oligonucleotide can hybridize to the *Chlamydia trachomatis* nucleic acid, if present in the test sample; and detecting any oligonucleotide hybridized to the *Chlamydia trachomatis* nucleic acid, wherein detection of the oligonucleotide hybridized to the *Chlamydia trachomatis* nucleic acid indicates the presence of *Chlamydia trachomatis* in the test sample.

In certain embodiments, the oligonucleotide is an oligonucleotide amplification primer of an inventive primer set. In other embodiments, the oligonucleotide is an oligonucleotide amplification primer or an oligonucleotide detection probe of an inventive primer/probe set.

In certain embodiments, the step of detecting comprises amplifying all or part of the *Chlamydia trachomatis* nucleic acid to obtain *Chlamydia trachomatis* amplicons, and detecting any *Chlamydia trachomatis* amplicons.

### Sample Preparation

The presence of *Chlamydia trachomatis* in a test sample can be determined by detecting any *Chlamydia trachomatis* nucleic acid comprising a sequence within the cryptic plasmid of *Chlamydia trachomatis.* Thus, any liquid or solid biological material suspected of comprising such *Chlamydia trachomatis* target sequences can be a suitable test sample. Preferred test samples include urine (*e.g.,* first void urine), seminal fluid, saliva, ocular lens fluid, lymphatic fluid, endocervical, urethral, rectal, vaginal, vulva-vaginal, and nasopharyngeal samples.

Test samples can be obtained or isolated from patients suspected of being infected with *Chlamydia trachomatis.* As already mentioned, a test sample may be used without further treatment/processing after isolation or, alternatively, it may be processed before analysis. For example, a test sample may be treated so as to release nucleic acids from any *Chlamydia trachomatis* cells that it may contain. Methods of nucleic acid extraction are well-known in the art and include chemical methods, temperature methods, and mechanical methods (see, for example, J. Sambrook et al., "Molecular Cloning: A Laboratory Manual", 1989, 2nd Ed., Cold Spring Harbour Laboratory Press: New York, NY). There are also numerous different and versatile kits that can be used to extract nucleic acids from biological samples that are commercially available from, for example, Amersham Biosciences (Piscataway, NJ), BD Biosciences Clontech (Palo Alto, CA), Epicentre Technologies (Madison, WI), Gentra Systems, Inc. (Minneapolis, MN), MicroProbe Corp. (Bothell, WA), Organon Teknika (Durham, NC), and Qiagen Inc. (Valencia, CA). User Guides that describe in great detail the protocol to be followed are usually included in these kits. Sensitivity, processing time and cost may be different from one kit to another. One of ordinary skill in the art can easily select the kit(s) most appropriate for a particular situation.

Prior to extraction, *Chlamydia trachomatis* cells may be purified, concentrated or otherwise separated from other components of the original biological sample, for example, by filtration or centrifugation.

### Sample Analysis

As will be appreciated by one skilled in the art, amplification of *Chlamydia trachomatis* target sequences and detection of amplified *Chlamydia trachomatis* nucleic acids according to the inventive methods may be performed using any amplification/detection methodologies described herein. In certain preferred embodiments, detection of *Chlamydia trachomatis* in a test sample is performed using a TaqMan™ assay, and the formation of amplification products is monitored in a real time manner by fluorescence. In these embodiments, probes are used that are labeled with a fluorescent reporter at the 5' end and a quencher moiety at the 3' end, as described above. Optimization of amplification conditions and selection of amplification reaction reagents suitable for a TaqMan™ assay format are within the skill in the art.

In certain embodiments, an internal control or an internal standard is added to the biological sample (or to purified nucleic acids extracted from the biological sample) to serve as a control for extraction and/or target amplification. Preferably, the internal control includes a sequence that differs from the target sequence(s), and is capable of amplification by the primers used to amplify the target *Chlamydia trachomatis* nucleic acids. The use of an internal control allows monitoring of the extraction process, amplification reaction, and detection, and control of the assay performance. The amplified control and amplified target are typically distinguished at the detection step by using different probes (*e.g.,* labeled with different detectable agents) for the detection of the control and the target.

The presence of *Chlamydia trachomatis* in a test sample may be confirmed by repeating an assay according to the present invention using a different aliquot of the same biological test sample or using a different test sample (*e.g.,* an endocervical swab if the first sample analyzed was a urine sample, or a urine sample collected at a different time). Alternatively or additionally, the presence of *Chlamydia trachomatis* in a test sample may be confirmed by performing a different assay (*i.e.,* an assay based on a different methodology). For example, if the first analysis was performed using a TaqMan™ assay, a second analysis may be carried out using a transcription-mediated amplification (TMA) reaction.

Alternatively, the presence of *Chlamydia trachomatis* in a test sample may be confirmed by a non-inventive assay.

### III - Simultaneous Detection of Chlamydia trachomatis and Other Organisms

As already mentioned, the primer/probe sets of the present invention are specific for *Chlamydia trachomatis.* The present Applicants have challenged the Primer/Probe Set CT5 in a multiplex assay format with 74 closely related organisms listed in Table 4 and found no cross-correlation (see Example 1).

Accordingly, the present invention also provides methods for simultaneously detecting the presence of *Chlamydia trachomatis* and another organism in a test sample using a combination of at least two primer/probe sets (*i.e.,* one selected from the *Chlamydia trachomatis* specific primer/probe sets disclosed herein and another selected from primer/probe sets specific for the other organism to be tested).

Other organisms that can be detected simultaneously with *Chlamydia trachomatis* include, but are not limited to, any of the organisms listed in Table 4. In certain embodiments, the other organism is *Neisseria gonorrhea.*

In particular, the present invention provides a method for the detection of *Chlamydia trachomatis* and/or *Neisseria gonorrhea* in a test sample, which comprises steps of: providing a test sample suspected of containing a *Chlamydia trachomatis* nucleic acid and/or a *Neisseria gonorrhea* nucleic acid; contacting the test sample with Primer/Probe Set CT(mpx) such that at least one of the primers or probes of the Primer/Probe Set CT(mpx) can hybridize to the *Chlamydia trachomatis* nucleic acid, if present in the test sample; contacting the test sample with at least one primer/probe set specific for *Neisseria gonorrhea* such that at least one of the primers or probes of the primer/probe set specific for *Neisseria gonorrhea* can hybridize to the *Neisseria gonorrhea* nucleic acid, if present in the test sample; detecting any primer or probe of the Primer/Probe Set CT(mpx) hybridized to the *Chlamydia trachomatis* nucleic acid, where the detection of a primer or probe hybridized to the *Chlamydia trachomatis* nucleic acid indicates the presence of *Chlamydia trachomatis* in the test sample; and detecting any primer or probe of the primer/probe set specific for *Neisseria gonorrhea* hybridized to the *Neisseria gonorrhea* nucleic acid, where the detection of a primer or probe hybridized to the *Neisseria gonorrhea* nucleic acid indicates the presence of *Neisseria gonorrhea* in the test sample. In certain embodiments, a primer/probe set specific for *Neisseria gonorrhea* is selected from the primer/probe sets described in Provisional Application No. 60/790,197 filed on April 7, 2006 and entitled *"Neisseria gonorrhoeae* Specific Oligonucleotide Sequences".

### IV - Kits

In another aspect, the present invention provides kits comprising materials useful for the detection of chlamydia according to methods described herein. The kits may be used by diagnostic laboratories, experimental laboratories, or practitioners.

Basic materials and reagents required for the detection of *Chlamydia trachomatis* according to the present invention may be assembled together in a kit. In certain embodiments, the kit comprises at least one primer set or primer/probe set, and optionally, amplification reaction reagents. Each kit preferably comprises the reagents which render the procedure specific. Thus, a kit adapted for use with NASBA preferably contains primers with an RNA polymerase promoter linked to the target binding sequence, while a kit adapted for use with SDA preferably contains primers including a restriction endonuclease recognition site 5' to the target binding sequence. Similarly, when the kit is adapted for use in a 5' nuclease assay, such as the TaqMan™ assay, the detection probes preferably contain at least one fluorescent reporter moiety and at least one quencher moiety.

Suitable amplification reaction reagents include, for example, one or more of: buffers, reagents, enzymes having reverse transcriptase and/or polymerase activity or exonuclease activity; enzyme cofactors such as magnesium or manganese; salts; nicotinamide adenide dinuclease (NAD); and deoxynucleoside triphosphates (dNTPs) such as, for example, deoxyadenosine triphospate; deoxyguanosine triphosphate, deoxycytidine triphosphate and thymidine triphosphate suitable for carrying out the amplification reaction. For example, a kit, adapted for use with NASBA, may contain suitable amounts of reverse transcriptase, RNase H and T7 RNA polymerase. In kits adapted for transcription amplification reactions, such as NASBA, buffers can be included that contain, for example, DMSO, which is known to enhance the amplification reaction.

Depending on the procedure, the kit may further comprise one or more of: wash buffers and/or reagents, hybridization buffers and/or reagents, labeling buffers and/or reagents, and detection means. The buffers and/or reagents included in a kit are preferably optimized for the particular amplification/detection technique for which the kit is intended. Protocols for using these buffers and reagents for performing different steps of the procedure may also be included in the kit.

Furthermore, the kits may be provided with an internal control as a check on the amplification procedure and to prevent occurrence of false negative test results due to failures in the amplification procedure. An optimal control sequence is selected in such a way that it will not compete with the target nucleic acid sequence in the amplification reaction (as described above).

Kits may also contain reagents for the isolation of nucleic acids from biological specimen prior to amplification and/or for the purification or separation of *Chlamydia trachomatis* cells before nucleic acid extraction.

The reagents may be supplied in a solid (*e.g*., lyophilized) or liquid form. The kits of the present invention optionally comprise different containers (*e.g*., vial, ampoule, test tube, flask or bottle) for each individual buffer and/or reagent. Each component will generally be suitable as aliquoted in its respective container or provided in a concentrated form. Other containers suitable for conducting certain steps of the amplification/detection assay may also be provided. The individual containers of the kit are preferably maintained in close confinement for commercial sale.

The kit may also comprise instructions for using the amplification reaction reagents and primer sets or primer/probe sets according to the present invention. Instructions for using the kit according to one or more methods of the invention may comprise instructions for processing the biological sample, extracting nucleic acid molecules, and/or performing the test; instructions for interpreting the results as well as a notice in the form prescribed by a governmental agency (*e.g.,* FDA) regulating the manufacture, use or sale of pharmaceuticals or biological products.

### Examples

The following example describes some of the preferred modes of making and practicing the present invention. However, it should be understood that this example is for illustrative purposes only and is not meant to limit the scope of the invention. Furthermore, unless the description in the Example is presented in the past tense, the text, like the rest of the specification, is not intended to suggest that experiments were actually performed or data were actually obtained.

### Example 1: Specificity of Chlamydia trachomatis/Neisseria gonorrhea Multiplex Assay

A multiplex TaqMan kPCR assay was used to test fifteen (15) different *Chlamydia trachomatis* (CT) serovars (*i.e.,* A, B, Ba, C, D, E, F, G, H, I, J, K, L1, L2 and L3) and forty-six (46) different *Neisseria gonorrhea* (GC) isolates.

The amplification and detection in a single, sealed reaction well was carried out using Stratagene's Mx3000*P*™ Real-Time PCR System (Stratagene Inc., San Diego, CA). The assay master mix used in these experiments contained Taq DNA Polymerase, buffer, reference dye (ROX), and MgCl₂, AmpErase™ UNG (1 units/µL), from Applied Biosystems (Perkin-Elmer Applied Biosystems, Foster City, CA) or QIAGEN (Hilden, Germany); TaqMan® oligonucleotide primers and probes were synthesized in-house or purchased from BioSearch Inc. The kPCR reaction mix was comprised of 25 µL of master mix and 25 µL of purified DNA.

The results obtained are reported in Table 3. These results show that the CT/GC multiplex assay can detect a broad rage of CT serovars and GC isolates.

The CT/GC multiplex PCR master mix was also challenged with 10⁷ copies of genomic DNA from 74 closely related organisms (listed in Table 4), and showed no cross-reactivity.

## Claims

1. Use of an oligonucleotide defined by a nucleic acid sequence according to SEQ ID NO: 1 for detecting *Chlamydia trachomatis* in a test sample, wherein the step of detecting comprises contacting the test sample with the nucleic acid sequence according to SEQ ID NO: 1 such that the oligonucleotide can hybridize to the *Chlamydia trachomatis* nucleic acid and amplifying all or a portion of the *Chlamydia trachomatis* nucleic acid to obtain *Chlamydia trachomatis* amplicons, and detecting any *Chlamydia trachomatis* amplicons, wherein amplifying all or a portion of the *Chlamydia trachomatis* nucleic acid comprises submitting the test sample to a nucleic acid amplification reaction carried out under suitable amplification conditions and in the presence of suitable amplification reaction reagents.

2. The use of an oligonucleotide according to claim 1, wherein the oligonucleotide further comprises a detactable label.

3. The use of an oligonucleotide according to claim 2, wherein the detectable label is directly attached to the oligonucleotide.

4. The use of an oligonucleotide according to claim 2, wherein the detectable label is indirectly attached to the oligonucleotide.

5. The use of an oligonucleotide according to claim 2, wherein the detectable label is directly detectable.

6. The use of an oligonucleotide according to claim 2, wherein the detectable label is indirectly detectable.

7. The use of an oligonucleotide according to claim 2, wherein the detectable label comprises a fluorescent moiety attached at the 5' end of the oligonucleotide.

8. The use of an oligonucleotide according to claim 2, wherein said oligonucleotide further comprises a quencher moiety attached at the 3' end.

9. The use of an oligonucleotide according to claim 8, wherein the fluorescent moiety comprises 6-carboxyfluorescein and the quencher moiety comprises a dark quencher capable of quenching across the entire visible spectrum.

## Patentansprüche

1. Verwendung eines durch eine Nukleinsäuresequenz gemäß SEQ ID NO: 1 definierten Oligonukleotids zum Nachweisen von *Chlamydia trachomatis* in einer Testprobe, wobei der Nachweisschritt Inkontaktbringen der Testprobe mit der Nukleinsäuresequenz gemäß SEQ ID NO: 1, so dass das Oligonukleotid an die *Chlamydia trachomatis*-Nukleinsäure hybridisieren kann, und Amplifizieren der gesamten *Chlamydia trachomatis*-Nukleinsäure oder eines Teils davon unter Erhalt von *Chlamydia trachomatis*-Amplifikaten und Nachweisen jeglicher *Chlamydia trachomatis*-Amplifikate umfasst, wobei Amplifizieren der gesamten *Chlamydia trachomatis-*Nukleinsäure oder eines Teils davon umfasst, dass die Testprobe einer Nukleinsäureamplifikationsreaktion unterzogen wird, die unter geeigneten Amplifikationsbedingungen und in Gegenwart geeigneter Amplifikationsreaktionsreagentien durchgeführt wird.

2. Verwendung eines Oligonukleotids nach Anspruch 1, wobei das Oligonukleotid ferner eine nachweisbare Markierung umfasst.

3. Verwendung eines Oligonukleotids nach Anspruch 2, wobei die nachweisbare Markierung direkt an das Oligonukleotid gebunden ist.

4. Verwendung eines Oligonukleotids nach Anspruch 2, wobei die nachweisbare Markierung indirekt an das Oligonukleotid gebunden ist.

5. Verwendung eines Oligonukleotids nach Anspruch 2, wobei die nachweisbare Markierung direkt nachweisbar ist.

6. Verwendung eines Oligonukleotids nach Anspruch 2, wobei die nachweisbare Markierung indirekt nachweisbar ist.

7. Verwendung eines Oligonukleotids nach Anspruch 2, wobei die nachweisbare Markierung eine am 5'-Ende des Oligonukleotids gebundene Fluoreszenzgruppierung umfasst.

8. Verwendung eines Oligonukleotids nach Anspruch 2, wobei das Oligonukleotid ferner eine am 3'-Ende gebundene Quencher-Gruppierung umfasst.

9. Verwendung eines Oligonukleotids nach Anspruch 8, wobei die Fluoreszenzgruppierung 6-Carboxyfluorescein umfasst und die Quencher-Gruppierung einen dunklen Quencher mit der Fähigkeit zum Quenching über das gesamte sichtbare Spektrum umfasst.

## Revendications

1. Utilisation d'un oligonucléotide défini par une séquence d'acide nucléique selon SEQ ID N° 1 pour détecter *Chlamydia trachomatis* dans un échantillon d'essai, dans laquelle l'étape de détection comprend la mise en contact de l'échantillon d'essai avec la séquence d'acide nucléique selon SEQ ID N° 1 de telle sorte que l'oligonucléotide puisse s'hybrider avec l'acide nucléique de *Chlamydia trachomatis* et amplifier tout ou partie de l'acide nucléique de *Chlamydia trachomatis* pour obtenir des amplicons de *Chlamydia trachomatis,* et la détection de tout amplicon de *Chlamydia trachomatis,* dans laquelle l'amplification de tout ou partie de l'acide nucléique de *Chlamydia trachomatis* comprend la soumission de l'échantillon d'essai à une réaction d'amplification d'acide nucléique réalisée dans des conditions d'amplification adéquates et en présence de réactifs de réaction d'amplification adéquats.

2. Utilisation d'un oligonucléotide selon la revendication 1, dans laquelle l'oligonucléotide comprend en outre un marqueur détectable.

3. Utilisation d'un oligonucléotide selon la revendication 2, dans laquelle le marqueur détectable est directement attaché à l'oligonucléotide.

4. Utilisation d'un oligonucléotide selon la revendication 2, dans laquelle le marqueur détectable est indirectement attaché à l'oligonucléotide.

5. Utilisation d'un oligonucléotide selon la revendication 2, dans laquelle le marqueur détectable est directement détectable.

6. Utilisation d'un oligonucléotide selon la revendication 2, dans laquelle le marqueur détectable est indirectement détectable.

7. Utilisation d'un oligonucléotide selon la revendication 2, dans laquelle le marqueur détectable comprend une fraction fluorescente attachée à l'extrémité 5' de l'oligonucléotide.

8. Utilisation d'un oligonucléotide selon la revendication 2, dans laquelle ledit oligonucléotide comprend en outre une fraction d'extincteur attaché à l'extrémité 3'.

9. Utilisation d'un oligonucléotide selon la revendication 8, dans laquelle la fraction fluorescente comprend de la 6-carboxyfluorescéine et la fraction d'extincteur comprend un extincteur foncé capable d'opérer une extinction sur le spectre visible entier.
